# EUROPEAN PATENT SPECIFICATION

(11) **EP 4 094 772 B1**
(45) Date of publication and mention of the grant of the patent: **25.12.2024**
(21) Application number: 22175050.8
(22) Date of filing: 24.05.2022
(51) Int. Cl.: A61K 36/738, A61P 17/04, A61P 37/08, A23L 33/105

(54) **PHARMACEUTICAL COMPOSITION COMPRISING ROSE EXTRACT AS ACTIVE INGREDIENT FOR PREVENTING OR TREATING OF ALLERGY DISEASE**
PHARMAZEUTISCHE ZUSAMMENSETZUNG , DIE ROSENEXTRAKT ALS WIRKSTOFF ZUM VORBEUGEN ODER BEHANDELN VON ALLERGISCHEN ERKRANKUNGEN UMFASST
COMPOSITION PHARMACEUTIQUE COMPRENANT UN EXTRAIT DE ROSE EN TANT QU'INGRÉDIENT ACTIF POUR LA PRÉVENTION OU LE TRAITEMENT DE MALADIES ALLERGIQUES

(30) Priority: 25.05.2021 KR 20210066947; 25.04.2022 KR 20220050948
(43) Date of publication of application: 30.11.2022
(73) Proprietor: Designed Cells Co., Ltd., Cheongju-si, Chungcheongbuk-do 28576 (KR)
(72) Inventor: KIM, Yun Bae, 30092 Sejong-si (KR); CHOI, Ehn Kyoung, 34053 Daejeon (KR); KIM, Tae Myoung, 28498 Cheongju-si (KR)
(74) Representative: Eisenführ Speiser

(56) References cited:
- WO-A1-2018/016677
- WO-A1-2020/218781
- KR-A- 20210 056 289

## Description

### BACKGROUND OF THE INVENTION

### 1. Field of the invention

The present disclosure relates to a rose extract for use in the prevention or treatment of allergic disease and to a pharmaceutical composition comprising it as an active ingredient.

### 2. Description of the Prior Art

With the increasing opportunity of exposure to various allergens causative of immune hypersensitivity, such as house dust mites, yellow dust, fine dust, pet hair, pollen, food additives, etc., more and more modern people have recently been suffering from allergies. Among others, atopic dermatitis affects 10 to 20 % of children and 1 to 3 % of adults, and has been steadily increasing in prevalence.

Allergies are classified into types I, II, III, and IV according to their immune mechanisms, with the categorization of types I, II, and III to immediate hypersensitivity and type IV to delayed hypersensitivity. Among them, type I allergies, also called anaphylaxis-type allergies, for which IgE antibody is responsible, include bronchial asthma, atopic diseases, allergic rhinitis, allergic conjunctivitis, etc.

Action mechanisms classify drugs for clinical use of allergies into a degranulation inhibitor, a chemical transmitter action inhibitor, and a chemical transmitter synthesis inhibitor. Chemical transmitter action inhibitors and chemical transmitter synthesis inhibitors have a relatively clear mechanism of drug action, whereas degranulation inhibitors are obscure in action mechanism. In addition, these drugs may incur several side effects due to their long-term administration. In relation thereto, reference may be made to Korean Patent Publication No. 10-2019-0123384 A which discloses elaeocarpusin which inhibits the histamine release and degranulation of mast cells and the expression of inflammatory cytokines, along with the use of elaeocarpusin in treating allergic diseases.

Document WO 2020/218781 A1 relates to a functional composition containing an immortalized stem cell-derived exosome-rich culture medium and rosebud extract as active ingredients.

Document KR 2021 0056289 A deals with a pharmaceutical composition for the prevention or treatment of obesity or allergy containing rose extract as an active ingredient.

Document WO 2018/016677 A1 relates to a complex plant extract having allergic rhinitis, atopic dermatitis or chronic asthma ameliorating effect.

### SUMMARY OF THE INVENTION

The present disclosure aims to provide a use of a rose extract in treating allergic diseases.

In accordance with an aspect thereof, the present disclosure provides a pharmaceutical composition comprising a rose extract as an active ingredient for use in the prevention or treatment of an allergic disease, wherein the extract is obtained from at least one cultivar selected from the group consisting of Lover Shy, Red Perfume, Jaemina Red, Pretty Velvet and Hanggina.

In accordance with another aspect thereof, the present disclosure provides a health functional food comprising a rose extract as an active ingredient for use in the prevention or alleviation of an allergic disease, wherein the extract is obtained from at least one cultivar selected from the group consisting of Lover Shy, Red Perfume, Jaemina Red, Pretty Velvet and Hanggina.

The rose extracts from the cultivars Lover Shy, Red Perfume, Jaemina Red, Pretty Velvet and Hanggina according to the present disclosure can find advantageous applications in the treatment of allergic diseases as they are superb in anti-oxidative and anti-inflammatory activities, contain high contents of 1,2,3-benzenetriol compound which exhibits anti-allergic activity, inhibit the degranulation of mast cells, and significantly suppress the acute systemic allergy anaphylaxis shock and the topical allergy itching in mouse animal models.

### BRIEF DESCRIPTION OF THE DRAWINGS

The above and other aspects, features and advantages of the present disclosure will be more apparent from the following detailed description taken in conjunction with the accompanying drawings, in which:

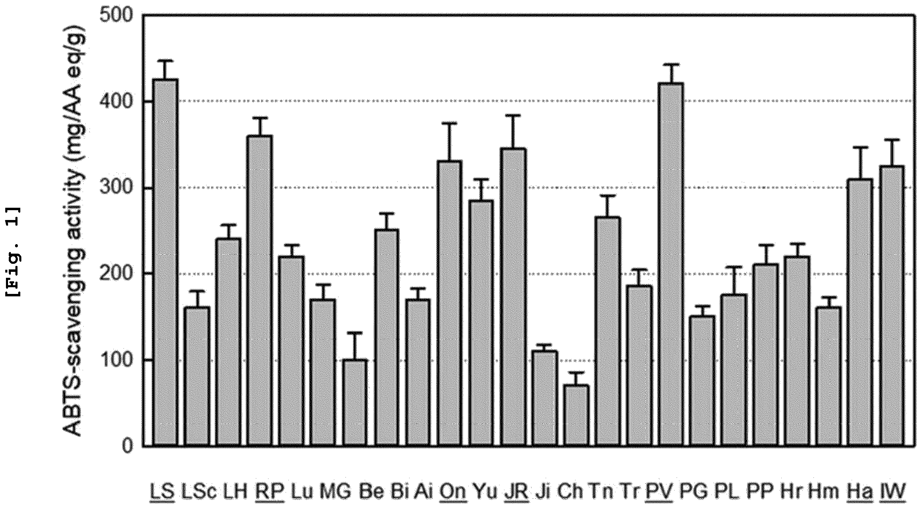

FIG. 1 is a graph of ABTS scavenging activities assayed from extracts from 24 rose cultivars, prepared in one Example of the present disclosure (LS: Love Shy, LSc: Lovely Scarlet, LH: Loving Heart, RP: Red Perfume, Lu: Luminus, MG: Mirinae Gold, Be: Betty, Bi: Bichina, Ai: Aileen, On: Onnuri, Yu: Yunina, JR: Jaemina Red, Ji: Jinseonmi, Ch: Chilbaegri, Tn: Tamina, Tr: Tamnari, PV: Pretty Velvet, PG: Peach Grace, PL: Pink Love, PP: Pink Perfume, Hr: Hanaro, Hm: Hanaram, Ha: Hanggina, IW: Ice Wing);

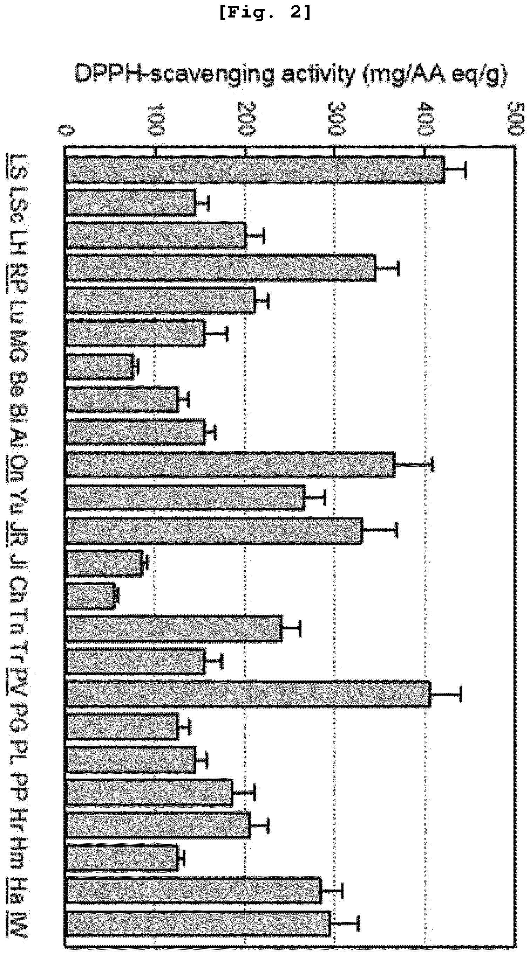

FIG. 2 is a graph of DPPH scavenging activities assayed from extracts from 24 rose cultivars, prepared in one Example of the present disclosure (LS: Love Shy, LSc: Lovely Scarlet, LH: Loving Heart, RP: Red Perfume, Lu: Luminus, MG: Mirinae Gold, Be: Betty, Bi: Bichina, Ai: Aileen, On: Onnuri, Yu: Yunina, JR: Jaemina Red, Ji: Jinseonmi, Ch: Chilbaegri, Tn: Tamina, Tr: Tamnari, PV: Pretty Velvet, PG: Peach Grace, PL: Pink Love, PP: Pink Perfume, Hr: Hanaro, Hm: Hanaram, Ha: Hanggina, IW: Ice Wing);

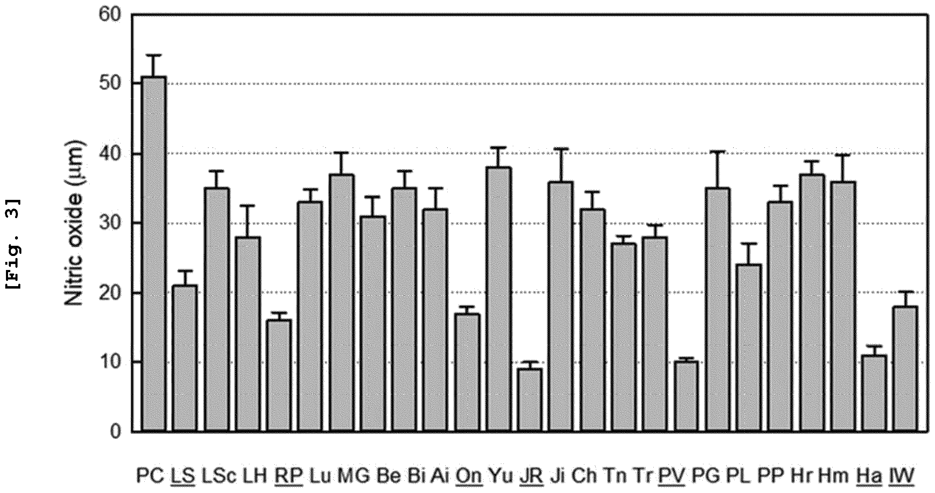

FIG. 3 is a graph of inhibitory activities of extracts from 24 rose cultivars, prepared in one Example of the present disclosure, against nitric oxide production (LS: Love Shy, LSc: Lovely Scarlet, LH: Loving Heart, RP: Red Perfume, Lu: Luminus, MG: Mirinae Gold, Be: Betty, Bi: Bichina, Ai: Aileen, On: Onnuri, Yu: Yunina, JR: Jaemina Red, Ji: Jinseonmi, Ch: Chilbaegri, Tn: Tamina, Tr: Tamnari, PV: Pretty Velvet, PG: Peach Grace, PL: Pink Love, PP: Pink Perfume, Hr: Hanaro, Hm: Hanaram, Ha: Hanggina, IW: Ice Wing);

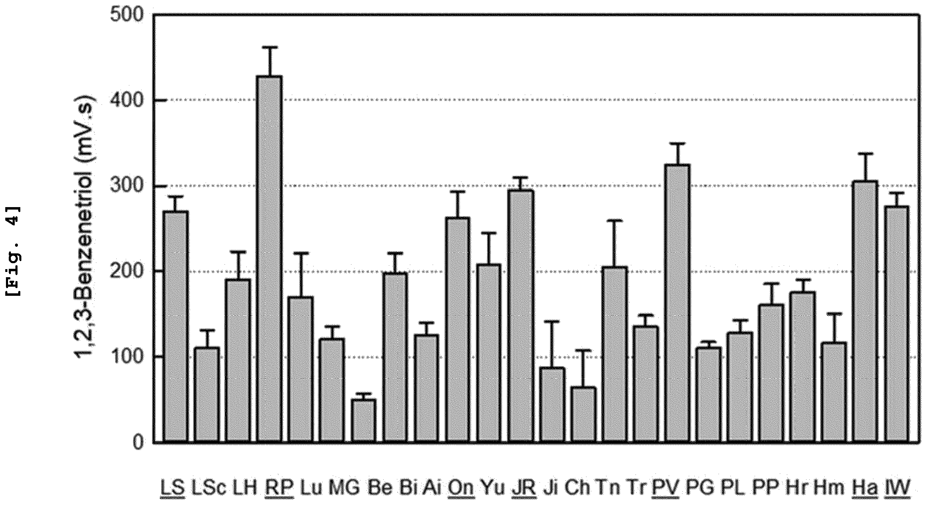

FIG. 4 is a graph of contents of 1,2,3-benzenetriol in extracts from 24 rose cultivars, prepared in one Example of the present disclosure (LS: Love Shy, LSc: Lovely Scarlet, LH: Loving Heart, RP: Red Perfume, Lu: Luminus, MG: Mirinae Gold, Be: Betty, Bi: Bichina, Ai: Aileen, On: Onnuri, Yu: Yunina, JR: Jaemina Red, Ji: Jinseonmi, Ch: Chilbaegri, Tn: Tamina, Tr: Tamnari, PV: Pretty Velvet, PG: Peach Grace, PL: Pink Love, PP: Pink Perfume, Hr: Hanaro, Hm: Hanaram, Ha: Hanggina, IW: Ice Wing); and

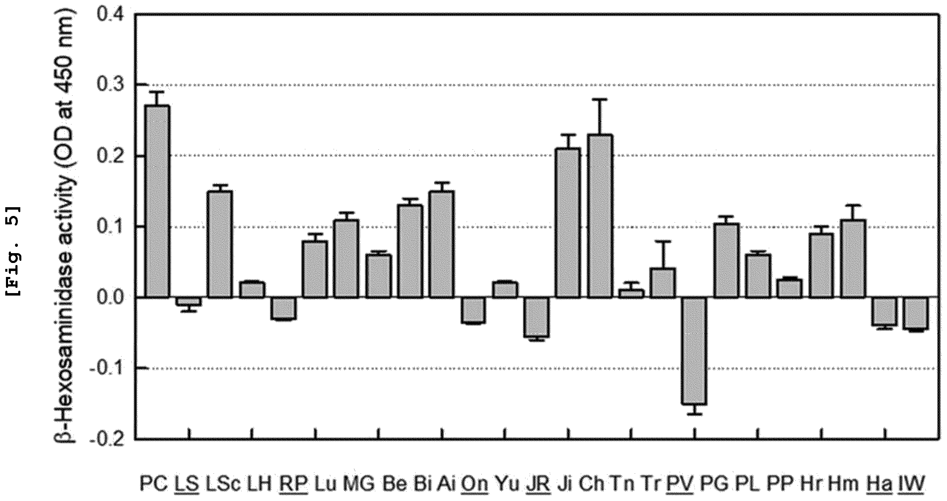

FIG. 5 is a graph of inhibitory activities of extracts from 24 rose cultivars, prepared in one Example of the present disclosure, against β-hexominidase release (LS: Love Shy, LSc: Lovely Scarlet, LH: Loving Heart, RP: Red Perfume, Lu: Luminus, MG: Mirinae Gold, Be: Betty, Bi: Bichina, Ai: Aileen, On: Onnuri, Yu: Yunina, JR: Jaemina Red, Ji: Jinseonmi, Ch: Chilbaegri, Tn: Tamina, Tr: Tamnari, PV: Pretty Velvet, PG: Peach Grace, PL: Pink Love, PP: Pink Perfume, Hr: Hanaro, Hm: Hanaram, Ha: Hanggina, IW: Ice Wing).

### DETAILED DESCRIPTION OF THE EXEMPLARY EMBODIMENTS

Below, a detailed description will be given of the present disclosure.

The present disclosure provides a pharmaceutical composition comprising a rose extract as an active ingredient for use in the prevention or treatment of an allergic disease, wherein the extract is obtained from at least one rose cultivar selected from the group consisting of Lover Shy, Red Perfume, Jaemina Red, Pretty Velvet and Hanggina.

The rose extract may be obtained from at least one selected from the group consisting of a petal, a stem, a leaf, a calyx, and a flower bud of rose and particularly from the group consisting of a petal, a calyx, and a flower bud of rose. In an embodiment of the present disclosure, the rose extract may be obtained from a flower bud.

The rose extract may be prepared by a preparation method comprising the followings steps of:
1) adding an extraction solvent to rose to afford an extract;
2) filtering the extract of step 1); and
3) concentrating the filtrate of step 2) at a reduced pressure, followed by drying the concentrate.

The extraction solvent may be water, an alcohol, or a combination thereof. The alcohol may be a lower alcohol of C₁ to C₂. In detail, the alcohol may be ethanol, methanol, or an alcohol spirit. The extraction solvent may be added in an amount 1- to 100-fold, 1- to 70-fold, 1- to 50-fold, 20- to 100-fold, 20- to 70-fold, 20- to 50-fold larger than the weight of the dried rose matter. The alcohol, when used as an extraction solvent, may range in alcohol concentration from 50 to 100%, from 50 to 90%, from 50 to 85%, from 70 to 100%, from 70 to 90%, or from 70 to 85%.

The extraction may be achieved using agitation extraction, cold precipitation extraction, reflux extraction, or ultrasound-assisted extraction. In this regard, the extraction may be conducted for 1 to 20 hours, 2 to 20 hours, 1 to 10 hours, 2 to 10 hours, 1 to 5 hours, or 2 to 5 hours. The extraction may be repeated once or more times.

In step 3), the concentration at a reduced pressure may be conducted using a vacuum decompression concentrator or a vacuum rotary evaporator. In addition, the drying may be decompression drying, vacuum drying, boil drying, spray drying, or freeze drying, and particularly freeze drying.

As used herein, the term "allergic disease", also known as hypersensitive reaction, refers to a condition in which an abnormal response of the immune system is caused during the sensitization of a specific antigen. Allergic diseases are classified into immediate hypersensitivity and delayed hypersensitivity in terms of the time taken to express symptoms and into types 1, 2, 3, and 4 according to immune mechanisms. In addition, the range which the allergic symptoms affect also serves as a criterion of division into systemic and localized allergic reactions. However, there are also allergic diseases that are difficult to classify according to such criteria or which account for combined types.

The allergic disease according to the present disclosure is a type 1 allergic disease, as defined in the appended claims. The term "type 1 allergy" refers to an allergic disease caused by the IgE antibody binding to an allergen and then to a receptor on basophils or mast cells wherein it triggers the release of inflammatory chemicals such as histamine or leukotriene. Type 1 allergy is also called immediate allergy or anaphylaxis allergy because it takes a short time to develop a symptom from contact with an antigen.

By way of example, type 1 allergic disease may include anaphylactic shock, bronchial asthma, allergic rhinitis, atopic dermatitis, urticaria, and pruritus. In the present disclosure, the type 1 allergic disease is anaphylactic shock or pruritus, as is defined in the appended claims.

The pharmaceutical composition for use according to the present disclosure may contain the active ingredient rose extract in an amount of 10 to 95 % by weight, based on the total weight thereof. In addition to this active ingredient, the pharmaceutical composition of the present disclosure may further comprise at least one ingredient having a similar or identical function to the active ingredient.

The pharmaceutical composition for use according to the present disclosure may include a carrier, a diluent, an excipient, or a combination thereof generally used in biological preparations. So long as it is suitable for delivering the composition into the body, any pharmaceutically acceptable carrier may be employed in the present disclosure. For details, reference may be made to Merck Index, 13th ed., Merck & Co. Inc., which discloses compounds, saline, sterilized water, Ringer's solution, dextrose solution, maltodextrin solution, glycerol, ethanol, or a mixture thereof. If necessary, a general additive such as an antioxidant, a buffer, and a bacteriostatic agent can be additionally added.

For formulating the composition, typical diluents or excipients, such as fillers, extenders, binders, wetting agents, disintegrants, and surfactants, may be used.

The composition for use according to the present disclosure may be formulated into oral or parenteral preparations. Oral formulations may be in solid or liquid forms. The solid formulations for oral administration may be tablets, pills, powders, granules, capsules or troches. These solid formulations are prepared by mixing the composition with one or more suitable excipients such as starch, calcium carbonate, sucrose, lactose, gelatin, or a mixture thereof. Further, the solid preparations may contain lubricants such as magnesium stearate and talc, etc. On the other hand, the liquid formulations may be suspensions, solutions for internal use, emulsions or syrups, and the above-mentioned formulations can contain excipients such as wetting agents, sweeteners, aromatics and preservatives.

Formulations for parenteral administration may include injections, suppositories, powders for respiratory inhalation, aerosols for spray, powders and creams. Examples of the injection include sterilized aqueous solutions, non-aqueous solutions, suspensions, and emulsions. For the non-aqueous solutions or suspensions, propylene glycol, polyethylene glycol, vegetable oil such as olive oil, and injectable ester such as ethylolate may be used as solvents.

The composition for use according to the present disclosure may be administered orally or parenterally. For parenteral administration, an intraperitoneal, an intrarectal, a subcutaneous, an intravenous, an intramuscular, or an intrathoracic route may be taken.

The composition may be administered at a pharmaceutically effective dose. The pharmaceutically effective dose may vary depending on types and severity of disease, drug activity, the time of administration, the route of administration, and the duration of treatment, drugs being used simultaneously therewith, etc. For a desired effect, however, the pharmaceutical composition according to the present disclosure may contain the active ingredient in an amount of 0.0001 to 1,000 mg/kg and particularly in an amount of 0.001 to 500 mg/kg. The administration may be made once or several times a day.

The composition for use according to the present disclosure may be administered alone or in combination with other therapeutic agents. In combination administration, the administration can be sequential or simultaneous.

Also, the present disclosure provides a health functional food comprising a rose extract as an active ingredient for use in the prevention or treatment of an allergic disease, wherein the extract is obtained from at least one cultivar selected from the group consisting of Lover Shy, Red Perfume, Jaemina Red, Pretty Velvet and Hanggina.

The rose extract contained as an active ingredient in the health functional food for use according to the present disclosure may be characterized as stated in the foregoing. For example, the rose extract may be obtained from at least one selected from the group consisting of a petal, a stem, a leaf, a calyx, and a flower bud of rose. The rose extract may be obtained using water, a lower alcohol of C₁ to C₂, or a combination thereof, wherein the lower alcohol of C₁ to C₂ may be ethanol, methanol, or an alcohol spirit.

Also, the allergic disease may be characterized as stated in the foregoing. The rose extract for use according to the present disclosure may be added to a food as it is or in mixture with other foods or food components. The content of the active ingredient may be determined according to the purpose of use and may range from 0.01 to 90 parts by weight, based on the 100 parts by weight of the entire health functional food.

No particular limitations are imparted to the form and type of the health functional food. Specifically, the health functional food can be in the form of tablets, capsules, powders, granules, liquids, and pills. The health functional food may additionally include various flavors, sweeteners, or natural carbohydrates. The sweeteners may be natural or synthetic. Examples of natural sweeteners includes thaumatin and a stevia extract. The synthetic sweetener may be exemplified by saccharin and aspartame. The natural carbohydrates may be monosaccharides, disaccharides, polysaccharides, oligosaccharides and glucose alcohols.

In addition to the additional ingredients mentioned above, the health functional food of the present disclosure may include nutrients, vitamins, minerals, flavors, colorants, pectic acid and its salts, alginic acid and its salts, organic acids, protective colloidal thickeners, pH regulators, stabilizers, preservatives, glycerin, alcohols, etc. All these ingredients may be added singly or together. The mixing ratio of those ingredients may range from 0.01 to 0.1 part by weight per 100 parts by weight of the composition of the present disclosure.

Below, a better understanding of the present disclosure may be obtained via the Examples which are set forth to illustrate, but are not to be construed to limit, the present disclosure.

### EXAMPLE 1: Preparation of Flower Bud Extract from Rose Cultivar Lover Shy

An extract from the novel cultivar Lover Shy bred in Korea was prepared as follows.

First, a dry matter of Lover Shy flower buds (the Gumi Floriculture Research institute of Gyeongsangbuk-do Agricultural Research & Extension Services, Korea) was pulverized and added to 80% ethanol in an ultrasonic water bath. The bath was heated at 60 to 70°C for 2 hours before ultrasonic extraction for 1 hour. In this regard, the extraction ratio was set forth to be 49:1 (980 ml of 80% ethanol: 20 g of dry rose flower bud). The extract solution thus obtained was cooled to room temperature, filtered, and concentrated to 50 brix using a rotary vacuum evaporator (N-N series, Eyela, Japan) to afford a flower bud extract from the rose cultivar Lover Shy.

### EXAMPLE 2: Preparation of Flow Bud Extract from Rose Cultivar Lovely Scarlet

A rose flower bud extract was prepared in the same condition and manner as in Example 1, with the exception that flower buds of Lovely Scarlet were used instead of those of Lover Shy.

### EXAMPLE 3: Preparation of Flow Bud Extract from Rose Cultivar Loving Heart

A rose flower bud extract was prepared in the same condition and manner as in Example 1, with the exception that flower buds of Loving Heart were used instead of those of Lover Shy.

### EXAMPLE 4: Preparation of Flow Bud Extract from Rose Cultivar Red Perfume

A rose flower bud extract was prepared in the same condition and manner as in Example 1, with the exception that flower buds of Red Perfume were used instead of those of Lover Shy.

### EXAMPLE 5: Preparation of Flow Bud Extract from Rose Cultivar Luminus

A rose flower bud extract was prepared in the same condition and manner as in Example 1, with the exception that flower buds of Luminus were used instead of those of Lover Shy.

### EXAMPLE 6: Preparation of Flow Bud Extract from Rose Cultivar Mirinae Gold

A rose flower bud extract was prepared in the same condition and manner as in Example 1, with the exception that flower buds of Mirinae Gold were used instead of those of Lover Shy.

### EXAMPLE 7: Preparation of Flow Bud Extract from Rose Cultivar Betty

A rose flower bud extract was prepared in the same condition and manner as in Example 1, with the exception that flower buds of Betty were used instead of those of Lover Shy.

### EXAMPLE 8: Preparation of Flow Bud Extract from Rose Cultivar Bichina

A rose flower bud extract was prepared in the same condition and manner as in Example 1, with the exception that flower buds of Bichina were used instead of those of Lover Shy.

### EXAMPLE 9: Preparation of Flow Bud Extract from Rose Cultivar Aileen

A rose flower bud extract was prepared in the same condition and manner as in Example 1, with the exception that flower buds of Aileen were used instead of those of Lover Shy.

### EXAMPLE 10: Preparation of Flow Bud Extract from Rose Cultivar Onnuri

A rose flower bud extract was prepared in the same condition and manner as in Example 1, with the exception that flower buds of Onnuri were used instead of those of Lover Shy.

### EXAMPLE 11: Preparation of Flow Bud Extract from Rose Cultivar Yunina

A rose flower bud extract was prepared in the same condition and manner as in Example 1, with the exception that flower buds of Yunina were used instead of those of Lover Shy.

### EXAMPLE 12: Preparation of Flow Bud Extract from Rose Cultivar Jaemina Red

A rose flower bud extract was prepared in the same condition and manner as in Example 1, with the exception that flower buds of Jaemina Red were used instead of those of Lover Shy.

### EXAMPLE 13: Preparation of Flow Bud Extract from Rose Cultivar Jinseonmi

A rose flower bud extract was prepared in the same condition and manner as in Example 1, with the exception that flower buds of Jinseonmi were used instead of those of Lover Shy.

### EXAMPLE 14: Preparation of Flow Bud Extract from Rose Cultivar Chilbaegri

A rose flower bud extract was prepared in the same condition and manner as in Example 1, with the exception that flower buds of Chilbaegri were used instead of those of Lover Shy.

### EXAMPLE 15: Preparation of Flow Bud Extract from Rose Cultivar Tamina

A rose flower bud extract was prepared in the same condition and manner as in Example 1, with the exception that flower buds of Tamina were used instead of those of Lover Shy.

### EXAMPLE 16: Preparation of Flow Bud Extract from Rose Cultivar Tamnari

A rose flower bud extract was prepared in the same condition and manner as in Example 1, with the exception that flower buds of Tamnari were used instead of those of Lover Shy.

### EXAMPLE 17: Preparation of Flow Bud Extract from Rose Cultivar Pretty Velvet

A rose flower bud extract was prepared in the same condition and manner as in Example 1, with the exception that flower buds of Pretty Velvet were used instead of those of Lover Shy.

### EXAMPLE 18: Preparation of Flow Bud Extract from Rose Cultivar Peach Grace

A rose flower bud extract was prepared in the same condition and manner as in Example 1, with the exception that flower buds of Peach Grace were used instead of those of Lover Shy.

### EXAMPLE 19: Preparation of Flow Bud Extract from Rose Cultivar Pink Love

A rose flower bud extract was prepared in the same condition and manner as in Example 1, with the exception that flower buds of Pink Love were used instead of those of Lover Shy.

### EXAMPLE 20: Preparation of Flow Bud Extract from Rose Cultivar Pink Perfume

A rose flower bud extract was prepared in the same condition and manner as in Example 1, with the exception that flower buds of Pink Perfume were used instead of those of Lover Shy.

### EXAMPLE 21: Preparation of Flow Bud Extract from Rose Cultivar Hanaro

A rose flower bud extract was prepared in the same condition and manner as in Example 1, with the exception that flower buds of Hanaro were used instead of those of Lover Shy.

### EXAMPLE 22: Preparation of Flow Bud Extract from Rose Cultivar Hanaram

A rose flower bud extract was prepared in the same condition and manner as in Example 1, with the exception that flower buds of Hanaram were used instead of those of Lover Shy.

### EXAMPLE 23: Preparation of Flow Bud Extract from Rose Cultivar Hanggina

A rose flower bud extract was prepared in the same condition and manner as in Example 1, with the exception that flower buds of Hanggina were used instead of those of Lover Shy.

### EXAMPLE 24: Preparation of Flow Bud Extract from Rose Cultivar Ice Wing

A rose flower bud extract was prepared in the same condition and manner as in Example 1, with the exception that flower buds of Ice Wing were used instead of those of Lover Shy.

### EXPERIMENTAL EXAMPLE 1: Assay for Total Polyphenol Content

A total polyphenol content in each of the 24 rose flower bud extracts prepared above was measured using the Folin-Ciocalteu's phenol reagent.

First, concentrations of the rose flower bud extracts of Examples 1 to 24 were each adjusted into 1 mg/ml. To 100 µl of each of the concentration-adjusted rose flower bud extracts was added 2 ml of 2% Na₂CO₃, followed by incubation for 3 minutes. Then, 100 ul of Folin-Ciocalteu's phenol reagent was added, followed by incubation for an additional 30 minutes. Afterward, absorbance at 750 nm was read of the reaction solutions. In this regard, 10-, 20-, 30-, 40-, or 50-fold dilutions of gallic acid (Sigma-Aldrich, USA) were used as standard materials to construct a calibration curve. From the absorbance measurements, contents of polyphenol were calculated as mg gallic acid per 1 g of the extract, and the results are summarized in Table 1, below.

**TABLE 1**

| Sample | Total Polyphenol Content (mg/g) |
|---|---|
| Example 1 | 385.5±0.8 |
| Example 2 | 162.7±0.5 |
| Example 3 | 233.710.1 |
| Example 4 | 335.212.4 |
| Example 5 | 221.112.4 |
| Example 6 | 203.2±1.6 |
| Example 7 | 177.0±0.3 |
| Example 8 | 237.8±1.2 |
| Example 9 | 222.612.0 |
| Example 10 | 316.2±0.8 |
| Example 11 | 245.5±0.7 |
| Example 12 | 295.2±0.1 |
| Example 13 | 128.012.1 |
| Example 14 | 111.7±1.4 |
| Example 15 | 223.3±0.5 |
| Example 16 | 209.5±3.2 |
| Example 17 | 372.5±1.5 |
| Example 18 | 194.8±2.0 |
| Example 19 | 196.213.1 |
| Example 20 | 192.213.1 |
| Example 21 | 203.8±0.3 |
| Example 22 | 212.6±0.1 |
| Example 23 | 296.6±1.3 |
| Example 24 | 338.4±1.3 |

As shown in Table 1, extracts of Example 1, 4, 10, 12, 17, 23, and 24 were measured to have significantly high total polyphenol contents among the 24 rose flower bud extracts. Particularly, Example 1 had the highest total polyphenol content which was about 3.5-fold greater than the content of Example 14.

### EXPERIMENTAL EXAMPLE 2: Antioxidant Activity

The flower bud extracts prepared above from the 24 cultivars were measured for antioxidant activity using 2,2-azino-bis(3-ethylbenzothiazoline 6-sulfonic acid) (ABTS) and 1,1-diphenyl-2-picrylhydrazyl (DPPH) scavenging assays.

### 2-1. ABTS radical scavenging assay

First, an ABTS radical cation solution (7 mM) was added to 2.45 mM potassium persulfate solution and sufficiently stirred at room temperature 12 to 16 hours in a light-tight condition. The resulting solution was diluted with distilled water to set an absorbance f 1.4 to 1.5 at 735 nm. To 1 ml of the diluted ABTS radical cation solution was added 50 µl of each of the 0.5 mg/ml extracts from Examples 1 to 24 while distilled water served as a control. After one hour, absorbances of the mixtures were read at 753 nm using a spectrophotometer. From the measurements, absorbance differences between the test groups and the control were calculated as mg of ascorbic acid (AA) per 1 g of the extracts. The results are depicted in FIG. 1.

As shown in FIG. 1, significantly high ABTS scavenging activities were found in the flower bud extracts of Examples 1, 4, 10, 12, 17, 23, and 24 among the extracts from the 24 rose cultivars.

### 2-2. DPPH radical scavenging assay

First, 0.8 ml of 0.2 mM DPPH (Sigma-Aldrich, U.S.A.) was added to 0.2 ml of each of the 0.1 mg/ml extracts from Examples 1 to 24 and incubated at room temperature for 30 minutes while distilled water was used as a control. Then, absorbance was read at 520 nm. Differences of the absorbance reads between the control and the test groups were calculated into mg of ascorbic acid per 1 g of the extracts, and the results are depicted in FIG. 2.

As shown in FIG. 2, significantly high DPPH scavenging activities were found in the flower bud extracts of Examples 1, 4, 10, 12, 17, 23, and 24 among the extracts from the 24 rose cultivars.

### EXPERIMENTAL EXAMPLE 3: Anti-Inflammatory Activity

The flower bud extracts from the 24 rose cultivars were assayed for anti-inflammatory activity in terms of inhibitory effect on the production of nitric oxide (NO).

First, RAW264.7 cells (KCLB No.40071, Korea Research Institute of Bioscience & Biotechnology, Korea) were seeded at a density of 1×10⁶ cells per well into 96-well plates and incubated overnight. The incubated cells were added with 1 ug/ml lipopolysaccharide (LPS) and treated with 100 ug/ml of each extract of Examples 1 to 24. After 24 hours of incubation, the same amount of Griess reagent (Promega, U.S.A.) was added. Ten minutes later, absorbance was read at 540 nm. In this regard, a standard calibration curve was constructed with sodium nitrite. On the standard calibration curve, concentrations of nitric oxide were calculated from the absorbance reads. The results of nitric oxide concentration are depicted in FIG. 3.

As shown in FIG. 3, significantly high inhibitory activities against nitric oxide production were found in the flower bud extracts of Examples 1, 4, 10, 12, 17, 23, and 24 among the extracts from the 24 rose cultivars.

### EXPERIMENTAL EXAMPLE 4: Detection of Anti-Allergic Ingredient

It was analyzed whether 1,2,3-benzenetriol, identified as an ingredient to have anti-allergic activity in previous studies, was present in the rose flower bud extracts prepared above.

Briefly, the experiment was performed using HP-5 column-equipped GC/MS system (5975C, Agilent Technologies, U.S.A.). After 10 µl of each of the extracts of Examples 1 to 24 was introduced into the split injector, data was imported from 35 to 250 m/z using a 1059 V detector. The compound 1,2,3-benzenetriol was identified based on a comparison of its retention times and mass spectra with those of standards from the Wiley 7N library data of the GC/MS system. Measured contents of 1,2,3-benzenetriol in the extracts are depicted in FIG. 4.

As shown in FIG. 4, significantly high contents of 1,2,3-benzenetriol were measured in the flower bud extracts of Examples 1, 4, 10, 12, 17, 23, and 24 among the extracts from the 24 rose cultivars.

### EXPERIMENTAL EXAMPLE 5: Anti-Allergic Activity

The flower bud extracts from the 24 rose cultivars, prepared above, were assayed for anti-allergic activity in terms of inhibitory effect on degranulation.

First, the mast cell line RBL-2H3 (KCLB No.22256, Korea Research Institute of Bioscience & Biotechnology, Korea) was cultured in MEM (minimum essential medium) supplemented with 10% FBS (fetal bovine serum) and 1% antibiotic-antimycotic (GIBCO, U.S.A.) in a T75 flask at 37°C under a 5% CO₂ condition. The culture was treated with trypsin-EDTA to collect the cells which was then centrifuged at 500 × g for 5 minutes. The supernatant was decanted and the cell pellet was suspended in a culture medium. The suspension was diluted to a density of 2×10⁶ cells/ml and then seeded at a density of 2×10⁴ cells/well into 96-well plates. After stabilization for 3 hours, the culture medium was aspirated from each well to which a dilution of 10 ng/ml anti-DNP (dinitrophenyl)-IgE in a culture medium was then added. The cells were incubated overnight in the same condition. Separately, the rose flower bud extracts of Examples 1 to 24 were each serially diluted to 100, 32, 10, or 3.2 ug/ml in an extracellular buffer. The incubated cells were washed twice with 100 ul of PBS and added with 100 ul of each of the extract dilutions in the buffer. After incubation for 30 minutes in the same condition as above, 10 ug/ml HAS (DNP-human serum albumin) was added and incubated for an additional 1 hour in the same condition. Thereafter, 60 µl of the culture was mixed and reacted with 15 µl of a substrate buffer at 37°C for 2 hours. The reaction was terminated by adding 150 µl of a stop solution. Each of the mixtures was taken in an amount of 200 µl and measured for absorbance at 405 nm. From the absorbance reads, β-hexominidase activity was calculated, and the results are depicted in FIG. 5.

As shown in FIG. 5, the anti-DNP-IgE-induced release of β-hexominidase was remarkably restrained to a normal level or less by the flower bud extracts of Examples 1, 4, 10, 12, 17, 23, and 24 among the extracts from the 24 rose cultivars.

### EXPERIMENTAL EXAMPLE 6: Inhibitory Activity against Systemic Allergic Response

The rose flower bud extracts of Examples 1, 4, 10, 12, 17, 23, and 24, identified above to have excellent biological activities, were assayed for inhibitory effect on the systemic allergic response anaphylactic shock.

In brief, 6-week-old male ICR mice (25 to 28 g) (Daehan BioLink Co. Ltd., Korea) were acclimated to the laboratory for about one week before use. Two of the mice were accommodated in each cage (220x200x145 mm) and raised in the conditions including a temperature of 23±2°C, a relative moisture of 55±10%, a room-ventilation rate of 12 air changes per hours, a lighting period of 12 hours, and an illumination intensity of 150 to 300 lux. Each of the rose flower bud extracts of Examples 1, 4, 10, 12, 17, 23, and 24 was diluted in physiological saline and intraperitoneally injected at a dose of 10, 30, or 100 mg/kg into the ICR mice. After 30 minutes, intraperitoneal injection of Compound 48/80 was made at the lethal dose 8 mg/kg to induce anaphylactic shock. Thereafter, the death rates of the mice were recorded for 60 minutes and are summarized in Table 2, below.

**TABLE 2**

| Sample | Dose (mg/kg) | Fatality (%) |
|---|---|---|
| Control (saline) | - | 10/10 (100) |
| Example 1 | 10 | 4/6 (66.6) |
| | 30 | 1/6 (16.7) |
| | 100 | 0/6 (0.0) |
| Example 4 | 10 | 5/6 (83.3) |
| | 30 | 1/6 (16.7) |
| | 100 | 0/6 (0.0) |
| Example 10 | 10 | 4/6 (66.6) |
| | 30 | 1/6 (16.7) |
| | 100 | 0/6 (0.0) |
| Example 12 | 10 | 6/6 (100) |
| | 30 | 0/6 (0.0) |
| | 100 | 0/6 (0.0) |
| Example 17 | 10 | 2/6 (33.3) |
| | 30 | 0/6 (0.0) |
| | 100 | 0/6 (0.0) |
| Example 23 | 10 | 6/6 (100) |
| | 30 | 1/6 (16.7) |
| | 100 | 0/6 (0.0) |
| Example 24 | 10 | 4/6 (66.6) |
| | 30 | 1/6 (16.7) |
| | 100 | 0/6 (0.0) |

As is understood from data of Table 2, each of the rose flower bud extracts of Examples 1, 4, 10, 12, 17, 23, and 24 suppressed anaphylactic shock-induced death in a dose-dependent manner. Among others, injection at a dose of 100 mg/kg of all of the extract completely prevented anaphylactic shock, allowing all the mice to be alive, with no observation of even a temporary shock symptom.

### EXPERIMENTAL EXAMPLE 7: Inhibitory Activity against Localized Allergic Response

The rose flower bud extracts of Examples 1, 4, 10, 12, 17, 23, and 24, identified above to have excellent biological activities, were assayed for inhibitory effect on the localized allergic response pruritus cutaneous.

In brief, each of the rose flower bud extracts of Examples 1, 4, 10, 12, 17, 23, and 24 was diluted in physiological saline and orally administered at a dose of 10, 30, or 100 mg/kg into the ICR mice prepared as described in Experimental Example 6. After 30 minutes, subcutaneous injection of Compound 48/80 (1 mg/ml) was made at a dose of 50 µl into a site between the opposite scapulars. In this regard, 50 ug of Compound 48/80 was injected per one injection site. The frequency of scratching was recorded for 60 minutes to measure the degree of itchiness, and the results are summarized in Table 3, below.

**TABLE 3**

| Sample | Dose (mg/kg) | No. of scratch (scratch/60 min) |
|---|---|---|
| Control (saline) | - | 65±13 |
| Example 1 | 10 | 43111 |
| | 30 | 34±11 |
| | 100 | 2317 |
| Example 4 | 10 | 36±12 |
| | 30 | 32±10 |
| | 100 | 24±11 |
| Example 10 | 10 | 48115 |
| | 30 | 33±11 |
| | 100 | 1816 |
| Example 12 | 10 | 40±14 |
| | 30 | 32±14 |
| | 100 | 26±10 |
| Example 17 | 10 | 35±15 |
| | 30 | 29±13 |
| | 100 | 2018 |
| Example 23 | 10 | 39111 |
| | 30 | 28±11 |
| | 100 | 19±7 |
| Example 24 | 10 | 41±11 |
| | 30 | 30±13 |
| | 100 | 22±9 |

As shown in Table 3, each of the rose flower bud extracts of Examples 1, 4, 10, 12, 17, 23, and 24 reduced the frequency of scratching on itchy spots in a dose-dependent manner. Among others, administration at a dose of 100 mg/kg of all of the extract reduced the frequency of scratching to a 1/3 level of the control. From these data, it was understood that the rose flower bud extracts according to the present disclosure were readily absorbed in the gut and reached the subcutaneous tissue to significantly suppress the allergic responses caused of the degranulation of mast cells.

This project (result) has been conducted as a local government-university cooperation-based regional innovation project with the support of the Korea Research Foundation (2021RIS-001) under the fund of the Ministry of Education in 2021 (2021RIS-001).

## Claims

1. Rose extract for use in the prevention or treatment of an allergic disease, wherein the rose extract is obtained from at least one rose cultivar selected from Lover Shy, Red Perfume, Jaemina Red, Pretty Velvet and Hanggina,
wherein the allergic disease is anaphylactic shock or pruritus.

2. The rose extract of claim 1, wherein the rose extract is obtained from at least one selected from the group consisting of a petal, a stem, a leaf, a calyx, and a flower bud of rose.

3. The rose extract of claim 1, wherein the rose extract is obtained with water, a lower alcohol of C₁ to C₂, or a combination thereof.

4. The rose extract of claim 3, wherein the lower alcohol of C₁ to C₂ is ethanol, methanol, or an alcohol spirit.

5. Functional food or food supplement for use in the prevention or alleviation of an allergic disease, wherein the allergic disease is anaphylactic shock or pruritus, the functional food or food supplement comprising a rose extract from at least one rose cultivar selected from Lover Shy, Red Perfume, Jaemina Red, Pretty Velvet and Hanggina.

6. Pharmaceutical composition for use in the prevention or treatment of an allergic disease, wherein the allergic disease is anaphylactic shock or pruritus, comprising a rose extract according to any of claims 1 to 4, and a pharmaceutically acceptable carrier.

## Patentansprüche

1. Rosenextrakt zur Verwendung bei der Prävention oder Behandlung einer allergischen Krankheit, wobei der Rosenextrakt aus mindestens einem aus Lover Shy, Red Perfume, Jaemina Red, Pretty Velvet und Hanggina ausgewählten Rosenkultivar gewonnen wird,
wobei es sich bei der allergischen Krankheit um anaphylaktischen Schock oder Pruritus handelt.

2. Rosenextrakt nach Anspruch 1, wobei der Rosenextrakt aus mindestens einem Bestandteil ausgewählt aus der aus Blütenblatt, Stängel, Blatt, Kelch und Blütenknospe der Rose bestehenden Gruppe gewonnen wird.

3. Rosenextrakt nach Anspruch 1, wobei der Rosenextrakt mit Wasser, einem niederen C₁- bis C₂-Alkohol oder einer Kombination davon gewonnen wird.

4. Rosenextrakt nach Anspruch 3, wobei es sich bei dem niederen C₁- bis C₂-Alkohol um Ethanol, Methanol oder ein Alkoholdestillat handelt.

5. Funktionelles Lebensmittel oder Nahrungsergänzungsmittel zur Verwendung bei der Prävention oder Linderung einer allergischen Krankheit, wobei es sich bei der allergischen Krankheit um anaphylaktischen Schock oder Pruritus handelt, wobei das funktionelle Lebensmittel oder Nahrungsergänzungsmittel einen Rosenextrakt aus mindestens einem aus Lover Shy, Red Perfume, Jaemina Red, Pretty Velvet und Hanggina ausgewählten Rosenkultivar umfasst.

6. Pharmazeutische Zusammensetzung zur Verwendung bei der Prävention oder Behandlung einer allergischen Krankheit, wobei es sich bei der allergischen Krankheit um anaphylaktischen Schock oder Pruritus handelt, umfassend einen Rosenextrakt gemäß einem der Ansprüche 1 bis 4 und einen pharmazeutisch unbedenklichen Träger.

## Revendications

1. Extrait de rose pour son utilisation dans la prévention ou le traitement d'une maladie allergique, dans lequel l'extrait de rose est obtenu à partir d'au moins un cultivar de rose choisi parmi Lover Shy, Red Perfume, Jaemina Red, Pretty Velvet et Hanggina,
dans lequel la maladie allergique est un choc anaphylactique ou un prurit.

2. Extrait de rose selon la revendication 1, dans lequel l'extrait de rose est obtenu à partir d'au moins un choisi dans le groupe constitué par un pétale, une tige, une feuille, un calice, et un bouton floral de rose.

3. Extrait de rose selon la revendication 1, dans lequel l'extrait de rose est obtenu avec de l'eau, un alcool inférieur de C₁ à C₂, ou une combinaison de ceux-ci.

4. Extrait de rose selon la revendication 3, dans lequel l'alcool inférieur de C₁ à C₂ est de l'éthanol, du méthanol, ou un spiritueux.

5. Aliment fonctionnel ou complément alimentaire pour son utilisation dans la prévention ou le soulagement d'une maladie allergique, dans lequel la maladie allergique est un choc anaphylactique ou un prurit, l'aliment fonctionnel ou le complément alimentaire comprenant un extrait de rose provenant d'au moins un cultivar de rose choisi parmi Lover Shy, Red Perfume, Jaemina Red, Pretty Velvet et Hanggina.

6. Composition pharmaceutique pour son utilisation dans la prévention ou le traitement d'une maladie allergique, dans laquelle la maladie allergique est un choc anaphylactique ou un prurit, comprenant un extrait de rose selon l'une quelconque des revendications 1 à 4, et un véhicule pharmaceutiquement acceptable.
